# EUROPÄISCHE PATENTSCHRIFT

(11) **EP 1 779 800 B1**
(45) Veröffentlichungstag und Bekanntmachung des Hinweises auf die Patenterteilung: **02.01.2008**
(21) Anmeldenummer: 05022643.0
(22) Anmeldetag: 18.10.2005
(51) Int. Cl.: A61B 19/00, A61C 19/00, A61L 2/00, A61L 2/18, A61L 2/20

(54) **Reinigungs- oder Pflegegerät für medizinische Instrumente**
Device for maintenance or cleaning of medical instruments
Dispositif de nettoyage et de soins d'appareils dentaires

(43) Veröffentlichungstag der Anmeldung: 02.05.2007
(73) Patentinhaber: W & H Dentalwerk Bürmoos GmbH, 5111 Bürmoos (AT)
(72) Erfinder: Helfenbein, Gerald, 5133 Gilgenberg (AT); Schaffarzick, Daniel, 5061 Elsbethen (AT)

(56) Entgegenhaltungen:
- EP-A- 0 732 083
- DE-A1- 19 913 943
- US-A- 5 723 090
- US-A- 6 007 686

## Beschreibung

Die vorliegende Erfindung betrifft ein Reinigungs- oder Pflegegerät für medizinische und dentalmedizinische Instrumente, insbesondere die Anschlussvorrichtung eines Reinigungs- oder Pflegegeräts oder einer Reinigungskassette eines Reinigungs- oder Pflegegeräts.

Ein derartiges Gerät ist aus der AT 400 105 B bekannt. Zum Anschluss eines zu reinigenden medizinischen Instruments sind am Gerät eine oder mehrere Anschlussvorrichtungen vorgesehen, auf die das Instrument aufgeschoben bzw. aufgesteckt wird. Alternativ kann ein zu reinigendes Instrument auch auf einer Anschlussvorrichtung einer Reinigungskassette, wie sie in der DE 31 17 264 offenbart ist, befestigt werden. Die Reinigungskassette wird anschließend in das Reinigungs- oder Pflegegerät eingesetzt. Die Anschlussvorrichtungen des Reinigungs- oder Pflegegeräts oder der Reinigungskassette sind mit einer oder mehreren Quellen von Medien verbunden bzw. verbindbar und leiten die Medien durch ihr Inneres in das zu reinigende Instrument weiter. Die Medien, zum Beispiel Druckluft, Reinigungs-, Desinfektions- oder Schmiermittel, werden dabei unter Druck in die zu reinigenden Instrumente gefördert. Es ist daher notwendig Vorkehrungen zu treffen, dass ein zu reinigendes Instrument während eines Reinigungs- oder Pflegeverfahrens durch den Druck nicht auf der Anschlussvorrichtung verschoben wird, so dass im Übertrittsbereich der Medien in die Instrumente Lecke entstehen und zumindest Teile der Medien austreten, oder dass das Instrument nicht gänzlich von der Anschlussvorrichtung getrieben wird.

Aus der Offenlegungsschrift DE 199 13 943 A1 ist ein Pflegegerät für Handstücke mit einem Gehäuse, das einen Pflegeraum umschließt, bekannt, wobei in dem Pflegeraum mehrere Anschlussvorrichtung zum Anschluss von Handstücken in einer etwa vertikalen Pflegestellung angeordnet sind, wobei sich eine Pflegemittel-Zuführungsleitung zu den Anschlussvorrichtungen erstreckt, die mit einem Antriebskanal der verbundenen Handstücke in Verbindung steht, und wobei die Anschlussvorrichtungen zwischen einer Pflegestellung und einer Bedienungsstellung, in der sie mit ihrem freien Ende in Richtung der Tür des Pflegeraums weisen, schwenkbar gelagert sind.

Aus dem Stand der Technik sind unterschiedliche Maßnahmen zur sicheren Befestigung des auf die Anschlussvorrichtung aufgesteckten Instruments bekannt. So ist bei einer bekannten Reinigungskassette die Anschlussvorrichtung so beschaffen, dass der Reibungswiderstand zwischen der Anschlussvorrichtung und dem aufgesteckten Instrument ausreichend hoch ist, so dass das Instrument nicht durch den Druck verschoben wird. Dies erhöht jedoch in nachteiliger Weise auch die zum Aufstecken des Instruments auf und zum Lösen des Instruments von der Anschlussvorrichtung benötigte Kraft, so dass eine derartige Anschlussvorrichtung für den Anwender erschwert bedienbar ist.

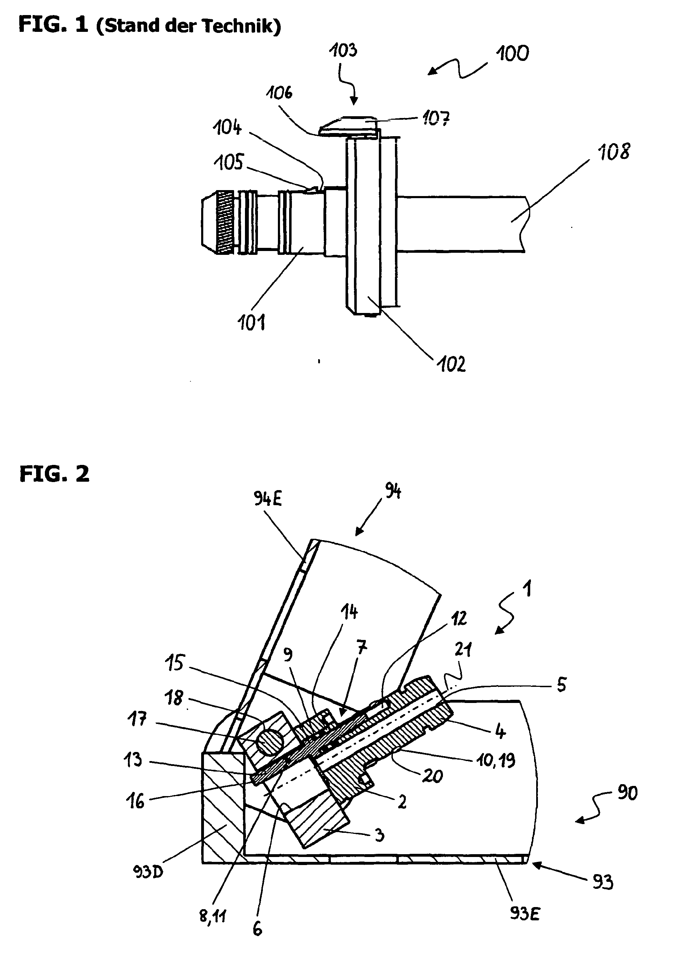

Bekannte Reinigungs- und Pflegegeräte mit Anschlussvorrichtungen für ein zu reinigendes Instrument weisen durch den Anwender zu bedienende Sperrmechanismen auf. Eine Ausführungsform eines derartigen Sperrmechanismus ist beispielhaft in Figur 1 als Teil einer bekannten Anschlussvorrichtung 100 dargestellt. Die Anschlussvorrichtung 100 umfasst einen Steckzapfen 101 auf den ein zu reinigendes Instrument aufgeschoben bzw. aufgesteckt wird, einen Flansch 102 und einen Sperrmechanismus 103. Über eine Leitung 108 werden der Anschlussvorrichtung 100 ein oder mehrere Medien zugeführt. Sperrmechanismus 103 besteht aus einem Sperrelement 104 mit einer Nase 105, einem Stift 106 und einem Druckknopf 107. Stift 106 ist in einer Bohrung des Flansches 102 aufgenommen und durch eine ebenfalls im Flansch 102 angeordnete Feder vorgespannt. Sperrelement 104 ist in einer Nut des Steckzapfens 101 aufgenommen und mit dem Stift 106 verbunden. Wird ein Instrument auf den Steckzapfen 101 geschoben, so schiebt sich eine Schulter des Kupplungsrohrs des Instruments über die Nase 105 und drückt die Nase 105 und das Sperrelement 104 in die Nut. Sobald die Schulter über die Nase 105 hinweg geglitten ist, kehrt Nase 105 wieder in ihre ursprüngliche, in der Figur 1 dargestellte Position, in der sie über die Oberfläche des Steckzapfens 101 hinausragt, zurück und hintergreift die Schulter des Instruments, wodurch dieses an der Anschlussvorrichtung fixiert ist. Zum Lösen des Instruments muss der Anwender Druckknopf 107 gegen die Federkraft drücken (in Figur 1 nach unten), wodurch Sperrelement 104 und Nase 105 in die Nut bewegt werden und, solange der Druckknopf 107 gedrückt bleibt, das Instrument vom Steckzapfen gezogen werden kann.

Nachteil dieser Art von Sperrmechanismen ist, dass sie vom Anwender bedient werden müssen, wobei der Druckknopf oftmals schwer zugänglich ist und meist beide Hände zum Bedienen des Druckknopfs und zur gleichzeitigen Entnahme des Instruments benötigt werden.

Der vorliegenden Erfindung liegt daher die Aufgabe zugrunde eine Anschlussvorrichtung zu schaffen, auf der die Instrumente sicher befestigbar sind, so dass sie während des Reinigungs- oder Pflegeverfahrens durch die unter Druck stehenden Medien nicht verschoben oder gelöst werden, und die ein unkompliziertes Anschließen und Lösen der Instrumente für den Anwender zulässt.

Diese Aufgabe wird gemäß der vorliegenden Erfindung durch ein Reinigungs- oder Pflegegerät oder eine Reinigungskassette mit einer Anschlussvorrichtung für medizinische Instrumente mit den Merkmalen des Anspruchs 1 und durch eine Anschlussvorrichtung mit den Merkmalen des Anspruchs 11 gelöst. Besonders vorteilhafte Ausführungsformen sind in den Unteransprüchen angeführt.

Bei dem erfindungsgemäßen Reinigungs-oder Pflesegerät nach Anspruch 1 ist eine direkte oder indirekte Wirkverbindung zwischen der Sperrvorrichtung der Anschlussvorrichtung und einem Bauteil des Reinigungs- oder Pflegegeräts oder zwischen der Sperrvorrichtung und zumindest einem Teil der Kassette vorgesehen, die die zur Bewegung der Sperrvorrichtung von der ersten Position in die zweite Position benötigte Kraft auf die Sperrvorrichtung überträgt.

Die Wirkverbindung kann als eine sich aufbauende oder kontinuierlich bestehende Wirkverbindung ausgebildet sein. Eine Wirkverbindung kann sich zum Beispiel während einer Handlung, die mit dem Reinigungs- oder Pflegeprozess oder Vorbereitungen dazu in Verbindung steht, insbesondere während des Beladens der Anschlussvorrichtung oder der Reinigungskassette mit den Instrumenten oder während des Einsetzens der Reinigungskassette in das Reinigungs- oder Pflegegerät, etablieren. Durch diese Handlung kommt es zu einer Relativbewegung und damit zu einer Annäherung eines Bauteils des Reinigungs- oder Pflegegeräts oder eines Teils der Kassette und der Anschlussvorrichtung und in Folge dieser Annäherung zum Aufbau der Wirkverbindung. Die Relativbewegung zwischen einem Bauteil des Reinigungs- oder Pflegegeräts oder einem Teil der Kassette und der Anschlussvorrichtung kann dabei als translatorische und / oder rotierende Bewegung ablaufen.

Die Wirkverbindung kann als berührungslose, bevorzugt magnetische Wirkverbindung, bei der zumindest Teile der Sperrvorrichtung und des Bauteils des Reinigungs- oder Pflegegeräts oder der Kassette magnetisch sind oder als eine kontaktierende Wirkverbindung, bei der das Bauteil des Reinigungs- oder Pflegegeräts oder ein Teil der Kassette die Sperrvorrichtung kontaktiert, ausgebildet sein.

Bei der Anschlussvorrichtung nach Anspruch 11 ermöglicht eine Verbindungsbohrung zwischen der Sperrvorrichtung und der Medienbohrung eine Ableitung eines Teils des in der Medienbohrung fließenden und unter Druck stehenden Mediums zur Sperrvorrichtung. Das Medium bewegt im Weiteren indirekt, zum Beispiel durch Verdrängen eines in einer Bohrung angeordneten Schiebeelements, oder direkt, zum Beispiel durch Verformen eines elastisch verformbaren Elements, die Sperrvorrichtung von der ersten in die zweite Position.

Als Instrumente im Sinne der vorliegenden Erfindung werden insbesondere Hand- und Winkelstücke zum Antrieb verschiedenster Werkzeuge, wie rotierender Bohrer, Feilen, Zahnsteinentfernungswerkzeuge, Sägen usw., Funktionshandstücke zur Abgabe von Wasser oder Luft und nicht angetriebene Handinstrumente, wie zum Beispiel medizinische Spiegel, Sonden und dergleichen verstanden.

Die Erfindung wird nachfolgend anhand von bevorzugten Ausführungsbeispielen und Bezug nehmend auf die beigefügten Zeichnungen erläutert:
Figur 1 zeigt eine aus dem Stand der Technik bekannte Anschlussvorrichtung mit einem Sperrmechanismus zum Fixieren des instruments.
Figur 2 zeigt ein erstes Ausführungsbeispiel der erfindungsgemäßen Anschlussvorrichtung, wobei die Anschlussvorrichtung drehbar in einer Reinigungskassette angeordnet ist und die Sperrvorrichtung für das Instrument durch die Kassette bewegt wird.
Figur 3 zeigt ein zweites Ausführungsbeispiel der erfindungsgemäßen Anschlussvorrichtung, bei der die Wirkverbindung zwischen der Sperrvorrichtung und der Wand des Reinigungs-oder Pflegegeräts besteht.
Figur 4 zeigt ein drittes Ausführungsbeispiel der erfindungsgemäßen Anschlussvorrichtung, bei der die Sperrvorrichtung ein schiefe Ebene und einen Fortsatz umfasst.
Figur 5 zeigt ein viertes Ausführungsbeispiel der erfindungsgemäßen Anschlussvorrichtung, bei der die Sperrvorrichtung einen Einstich und einen Sprengring umfasst, in einer Position in der das Instrument an der Anschlussvorrichtung fixiert ist.
Figur 6 zeigt die Anschlussvorrichtung von Figur 5 in einer Position, in der das Instrument auf die Anschlussvorrichtung aufschiebbar oder aufsteckbar bzw. von der Anschlussvorrichtung lösbar ist.
Figur 7 zeigt ein fünftes Ausführungsbeispiel der erfindungsgemäßen Anschlussvorrichtung, bei der die Sperrvorrichtung ein elastisch verformbares Element umfasst, in einer Position in der das Instrument an der Anschlussvorrichtung fixiert ist.
Figur 8 zeigt die Anschlussvorrichtung von Figur 7 in einer Position, in der das Instrument auf die Anschlussvorrichtung aufschiebbar oder aufsteckbar bzw. von der Anschlussvorrichtung lösbar ist.
Figur 9 zeigt ein sechstes Ausführungsbeispiel der erfindungsgemäßen Anschlussvorrichtung, bei der die Sperrvorrichtung über eine Verbindungsbohrung mit einer Medienbohrung verbunden ist.
Figur 10 zeigt eine Reinigungskassette mit einer erfindungsgemäßen Anschlussvorrichtung.

Die in Figur 2 dargestellte Anschlussvorrichtung 1 ähnelt in ihrem Aufbau einem standardisierten Motoransatz. Sie umfasst einen Anschlag 2, der die Einstecktiefe des Steckzapfens 4 eines auf die Anschlussvorrichtung 1 anzuschließendes Instruments, zum Beispiel eines Handstücks 91 oder eines Spiegels 92, begrenzt. Anschlag 2 ist auf einem Träger 3 einer Kassette 90 befestigt, wobei bevorzugt auf Träger 3 in bekannter Weise mehrere gleiche oder unterschiedliche Anschlussvorrichtungen 1 angeordnet sind. Der Steckzapfenzapfen 4 hat die selbe zylindrische Form und die selben Abmessungen wie ein standardisierter Kupplungszapfen eines Motors zum Antrieb eines Instruments.

Anschlussvorrichtung 1 ist von einer zentralen Medienbohrung oder Medienleitung 5 durchsetzt, die eine Quelle eines Reinigungs- oder Pflegemediums mit dem Inneren des aufgeschobenen oder aufgesteckten instruments verbindet. Selbstverständlich können auch weitere Leitungen und / oder Bohrungen vorgesehen sein, so dass das Instrument mit mehr als einem Medium gereinigt oder gepflegt werden kann. Medienleitung 5 setzt sich in einer Bohrung oder Leitung 6 durch Träger 3 fort. Wird die Reinigungskassette 90 in ein Reinigungs- oder Pflegegerät eingesetzt, so werden die Leitungen 5, 6 über eine Kupplung im Reinigungs- oder Pflegegerät und über Medienleitungen mit den Quellen der Medien verbunden.

Anschlussvorrichtung 1 umfasst des Weiteren eine Sperrvorrichtung 7 mit einem Schiebeelement 8, einem Federelement 9 und einem Sperrelement 10. Schiebeelement 8 ist als Stift 11 ausgebildet, der in einer Bohrung 12 der Anschlussvorrichtung 1 und in einer Bohrung 13 des Trägers 3 beweglich, insbesondere verschiebbar, aufgenommen ist. Stift 11 wird durch Federelement 9 in Form einer Spiralfeder vorgespannt. Federelement 9 ist in einer Kammer 14, deren Durchmesser größer ist als der Durchmesser der Bohrung 12, aufgenommen und drückt Flansch 15 des Stifts 11 gegen die Seitenwand der Kammer 14 bzw. des Trägers 3. Die Länge von Stift 11 ist so bemessen, dass in diesem entspannten Zustand des Federelements 9 Ende 16 von Stift 11 aus der Bohrung 12 bzw. 13 herausragt.

Sperrelement 10 in Form eines Sprengrings 19 ist in einer Ringnut 20 des Steckzapfens 4 angeordnet. Ein Durchstich in der Ringnut 20 verbindet die Ringnut 20 mit der darunter angeordneten Bohrung 12.

Anschlussvorrichtung 1 ist über Querachse 17 beweglich, d.h. dreh- oder schwenkbar, gelagert. Querachse 17 ist in einer Bohrung 18 in Träger 3 aufgenommen, selbstverständlich kann die Querachse jedoch auch in einer Bohrung der Anschlussvorrichtung 1 aufgenommen sein.

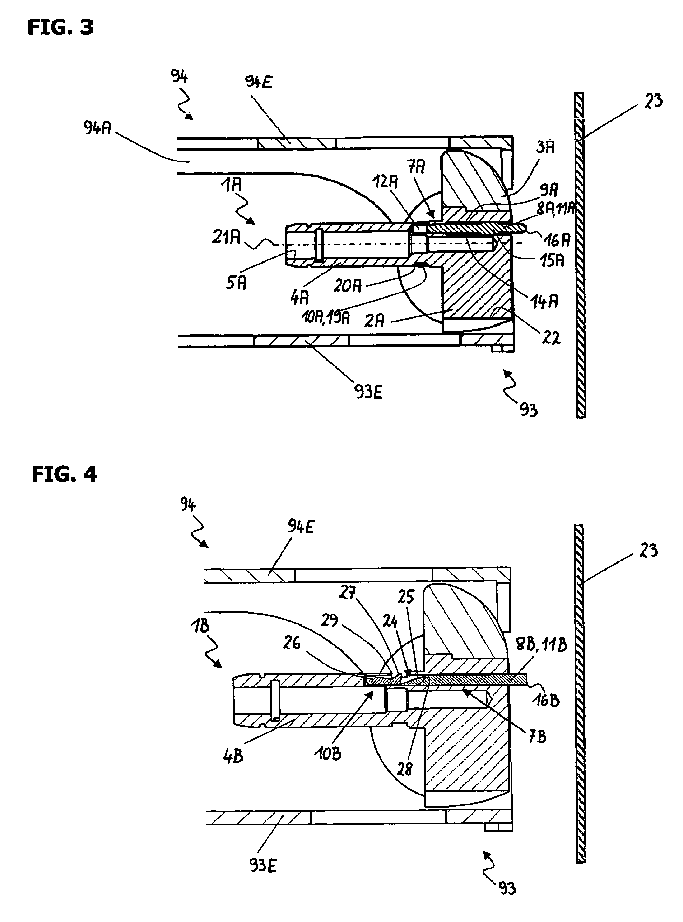

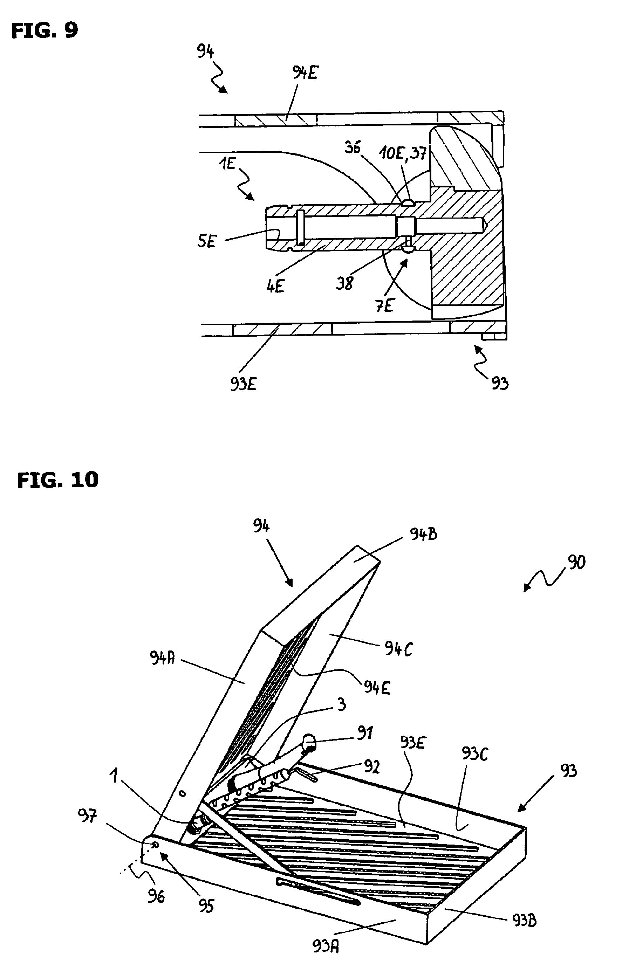

Wie insbesondere aus Figur 10 ersichtlich ist, besteht die Kassette 90 aus einem Basisteil 93 und einem Deckel 94, die über ein Scharnier 95 dreh- bzw. schwenkbar mit einander verbunden sind. Basisteil 93 und Deckel 94 sind rechteckig, wobei die Abmessungen des Deckels 94 etwas geringer sind als jene des Basisteils 93, so dass in geschlossenem, zusammengeklapptem Zustand Deckel 94 im Basisteil 93 aufgenommen ist. Basisteil 93 und Deckel 94 haben jeweils drei Seitenwände 93A, 93B, 93C und 94A, 94B, 94C. Bodenfläche 93E und Deckelfläche 94E sind durchbrochen, so dass die Reinigungs-oder Pflegemedien nach dem Durchtritt durch die Instrumente 91, 92 und / oder der Außenreinigung aus der Kassette 90 ablaufen können. In der Kassette 90 ist des Weiteren Träger 3 mit drei Anschlussvorrichtungen 1 aufgenommen. Um einen einfachen Anschluss der Anschlussvorrichtung 1 an die Kupplung im Reinigungs- oder Pflegegerät zu gewährleisten, fehlt eine vierten Seitenwand (Rückwand) vollständig (so wie in Figur 4 dargestellt) oder sie ist mit Durchbrechungen für die Kupplungen versehen. Die Rückwand kann als Teil des Deckels 94 oder, wie in Figur 2 dargestellt, als Teil des Basisteils 93 ausgeführt sein und ist entsprechend mit dem Bezugszeichen 93D benannt.

Schwenkt Anschlussvorrichtung 1 aus der in Figur 2 dargestellten Position um Querachse 17 in Richtung Rückwand 93D, so bewegt sich in einem ersten Teil der Relativbewegung Sperrvorrichtung 7 und Schiebeelement 8 mit Anschlussvorrichtung 1 mit, bis das Ende 16 des Schiebeelements 8 die der Anschlussvorrichtung 1 zugewandte Seite der Rückwand 93D kontaktiert. Dadurch entsteht eine kontaktierende Wirkverbindung im Sinne einer mechanischen Kopplung und in Form eines direkten Kontakts zwischen der Sperrvorrichtung 7 und der Rückwand 93D als Teil der Kassette 90. Wird Anschlussvorrichtung 1 weiter in Richtung Rückwand 93D geschwenkt, so überträgt die Wirkverbindung die zur Bewegung der Sperrvorrichtung 7 von der ersten Position in die zweite Position benötigte Kraft über die Rückwand 93D auf das Schiebeelement 8, so dass das Schiebeelement 8 seine in Figur 2 dargestellte Position verlässt und gegen die Kraft des Federelements 9 relativ zur Anschlussvorrichtung 1 in Richtung des Sprengrings 19 verschoben wird, bis es den Durchstich zwischen Bohrung 12 und Ringnut 20 erreicht.

Der in der Ringnut 20 angeordnete Sprengring 19 ragt in seiner Grundstellung in den Durchstich und in die Bohrung 12 hinein und steht nicht über die äußere Mantelfläche des Steckzapfens 4 hinaus. In dieser Position ist ein Instrument 91, 92 auf die Anschlussvorrichtung aufschiebbar oder aufsteckbar bzw. von der Anschlussvorrichtung lösbar. Wird Schiebeelement 8 unter den Durchstich verschoben, so verdrängt es Sprengring 19 - bezogen auf die Längsachse 21 des Steckzapfens 4 - radial nach außen, so dass Sprengring 19 über die äußere Mantelfläche des Steckzapfens 4 hinausragt. Ist ein Instrument 91, 92 auf den Steckzapfen 4 aufgesteckt, so drückt Sprengring 19 gegen ein Bauteil des Instruments 91, 92, insbesondere gegen das Kupplungsrohr, wodurch das Instrument 91, 92 gegen Verschieben oder Lösen gesichert ist.

Um das Instrument 91, 92, zum Beispiel nach Abschluss des Reinigungs- oder Pflegezyklus, wieder vom Steckzapfen 4 abnehmen zu können, muss Sprengring 19 wieder in seine ursprünglich Position überführt werden. Dies geschieht, in dem Anschlussvorrichtung 1 um Querachse 17 von Rückwand 93D weggeschwenkt wird, so dass die kontaktierende Wirkverbindung zwischen der Sperrvorrichtung 7, i.e. Schiebelement 8, und Rückwand 93D aufgehoben wird. Die durch das vorhergehende Verschieben von Schiebelement 8 gespannte Federelement 9 erhält so die Möglichkeit zu entspannen, wodurch Schiebeelement 8 über Flansch 15, an dem Federelement 9 angreift, in Richtung der Rückwand 93D verschoben wird und wieder die in Figur 2 dargestellt Position einnimmt. In dieser Position ist Schiebeelement 8 nicht mehr unter dem Durchstich angeordnet, so dass Sprengring 19 wieder in den Durchstich und in Bohrung 12 zurückkehrt und nicht mehr über die äußere Mantelfläche des Steckzapfens 4 hinausragt.

Das in Figur 2 dargestellt Ausführungsbeispiel, in dem Anschlussvorrichtung 1 drehbar an einer Querachse 17 angeordnet ist, kann anstelle in einer Reinigungskassette 90 in entsprechender Weise in einem Reinigungs- oder Pflegegerät implementiert sein. Rückwand 93D wird in diesem Fall von einem Bauteil des Reinigungs- oder Pflegegeräts ersetzt, insbesondere von der Rückwand des Innenraums des Reinigungs- oder Pflegegeräts.

In einem weiteren, nicht in einer Abbildung dargestellten Ausführungsbeispiel ist die Anschlussvorrichtung 1 gleich wie in Figur 2 aufgebaut, jedoch rotierbar um eine Längsachse angeordnet, wobei eine beliebige Längsachse, zum Beispiel Mittelachse 21 der Anschlussvorrichtung 1, gewählt werden kann, die jedoch nicht mit der Längsachse des Schiebeelements 8 ident sein darf. Die Anschlussvorrichtung 1 ist an einer Wand angeordnet, bevorzugt der Rückwand des Innenraums eines Reinigungs- oder Pflegegeräts. Rotiert die Anschlussvorrichtung 1 um die Längsachse 21 und somit relativ zur Rückwand, so durchläuft das Schiebeelement 8 eine kreis- oder kreisbogenförmige Bahn. Entlang dieser Bahn weist die Wand eine sich verändernde Wandstärke auf, die zum Beispiel durch eine sich vertiefende Nut oder eine schiefe Ebene gebildet wird. Befindet sich das durch ein Federelement 9 vorgespannte Schiebeelement 8 an der Stelle mit der geringsten Wandstärke, dann ragt es mit seinem Ende 16 in die Nut und nimmt eine wie in der Figur 2 dargestellte Position ein, so dass ein Instrument auf die Anschlussvorrichtung 1 aufgeschoben oder von der Anschlussvorrichtung 1 gelöst werden kann. Rotiert die Anschlussvorrichtung 1 um die Längsachse so wird das Schiebeelement 8 durch die zunehmende Wandstärke gegen die Federkraft des Federelements 9 in die Bohrung 12 der Anschlussvorrichtung 1 gedrückt, in der es wie für Figur 2 beschrieben mit dem Sperrelement 10 zusammenwirkt, um das Instrument 91, 92 an der Anschlussvorrichtung 1 zu fixieren. Zum Lösen der Instrumente 91, 92 wird Anschlussvorrichtung 1 so gedreht, dass sich Schiebeelement 8 wieder an eine Stelle mit einer geringen Wandstärke befindet.

Der Vorteil dieser Ausführungsform liegt insbesondere darin, dass für die Relativbewegung zwischen der Anschlussvorrichtung 1 und der Wand des Reinigungs- oder Pflegegeräts nur sehr wenig oder, wenn die Längsachse gleich der Mittelachse 21 der Anschlussvorrichtung 1 ist, kein Bewegungsraum benötigt wird. Dies ist insbesondere in Reinigungs- und Pflegegeräten mit ihren oft engen Innenräumen von Vorteil. Die Rotationsbewegung der Anschlussvorrichtung 1 kann entweder manuell durch den Anwender oder über Mitnehmervorrichtungen wie Zahnräder, Riementriebe oder Hebelarme erfolgen. In letzterem Fall ist die Mitnehmervorrichtung mit einem Betätigungsorgan verbunden, wobei das Betätigungsorgan bevorzugt durch die Tür des Reinigungs- und Pflegegeräts gebildet wird, so dass mit dem Schließen der Tür die auf der Anschlussvorrichtung 1 aufgesteckten Instrumente 91, 92 automatisch fixiert werden und mit dem Öffnen der Tür die Instrumente 91, 92 automatisch freigegeben werden bzw. Instrumente 91, 92 auf die Anschlussvorrichtung 1 aufgeschoben werden können.

Die in Figur 3 dargestellte Anschlussvorrichtung 1 A entspricht in ihrem Aufbau und in ihrer Funktionsweise Anschlussvorrichtung 1 aus Figur 2. Sie umfasst wiederum einen Steckzapfen 4A mit einer Medienleitung 5A und einem Anschlag 2A. Träger 3A ist in Form einer Leiste ausgeführt und umfasst eine oder mehrere Bohrungen 22 zur Aufnahme von einer oder mehreren Anschlussvorrichtungen 1A. Träger 3A und Anschlussvorrichtung 1A können fest oder drehbar, wie in Figur 10 dargestellt, in der Reinigungskassette 90 angeordnet sein, wobei die Drehbarkeit in dem Ausführungsbeispiel nach Figur 3 keine Voraussetzung für die Funktion der Sperrvorrichtung 7A ist. Ein drehbarer Träger 3A und eine drehbare Anschlussvorrichtung 1A bieten jedoch für den Anwender insbesondere eine deutlich komfortablere Möglichkeit des Aufsteckens oder Lösens der Instrumente 91, 92 auf die Anschlussvorrichtung 1 bzw. 1A. Sind Träger 3, 3A und / oder Anschlussvorrichtung 1, 1 A drehbar in einer Reinigungskassette 90 angeordnet, so ist es zum Zwecke einer einfachen Bauweise von Vorteil, wenn Träger 3, 3A und / oder Anschlussvorrichtung 1, 1 A und Deckel 94 eine gemeinsame Drehachse 96 haben, die bevorzugt durch eine einzige Welle 97 gebildet ist.

Sperrvorrichtung 7A umfasst ein Schiebeelement 8A in Form eines Kolbens 11 A mit einem Flansch 15A, eine Bohrung 12A zur Aufnahme des Schiebeelements 8A, ein in einer Kammer 14A aufgenommenes Federelement 9A und einen in einer Ringnut 20A angeordneten Sprengring 19A, der als Sperrelement 10A dient. Das Ver- und Entriegeln des Instruments 91, 92 erfolgt wie für Figur 2 beschrieben durch Verschieben des Schiebeelements 8A und von Sprengring 19A.

Im Unterschied zu dem Ausführungsbeispiel von Figur 2 erfolgt die Verschiebung des Schiebeelements 8A der Anschlussvorrichtung 1A durch ein Bauteil des Reinigungs- oder Pflegegeräts, insbesondere durch die Rückwand 23 des Innenraums des Reinigungs- oder Pflegegeräts, in das die Reinigungskassette 90 eingesetzt wird. Während des Einsetzens der Kassette 90 kommt es zu einer Annäherung der Kassette an das Bauteil, i.e. die Rückwand 23, bis Ende 16A von Kolben 11 A die Rückwand 23 kontaktiert, wodurch wiederum eine kontaktierende Wirkverbindung im Sinn einer mechanischen Kopplung entsteht. Bei einer weiteren Annäherung an die Rückwand 23 überträgt die Wirkverbindung die zum Verschieben benötigte Kraft auf das Schiebeelement 8A, so dass dieses bis zum Durchstich zwischen Bohrung 12A und Ringnut 20A verschoben wird. Wird die Kassette 90 von der Rückwand 23 entfernt und die Wirkverbindung damit unterbrochen, so zwingt Federelement 9A Schiebeelement 8A in die in Figur 3 dargestellt Ausgangsposition zurück.

Das in Figur 4 abgebildete Ausführungsbeispiel der Anschlussvorrichtung 1 B gleicht in Aufbau und Funktion weitgehend Anschlussvorrichtung 1A aus Figur 3. Auch hier erfolgt die Verriegelung des Instruments 91, 92 durch eine kontaktierende Wirkverbindung zwischen einem Bauteil des Reinigungs- oder Pflegegeräts, insbesondere der Rückwand 23 des Innenraums des Reinigungs- oder Pflegegeräts, und dem Ende 16B des als Stift 11 B ausgebildeten Schiebeelements 8B.

Im Unterschied zu Anschlussvorrichtung 1A aus Figur 3 ist das dem Ende 16B gegenüberliegenden Ende 24 des Schiebeelements 8B als schiefe Ebene 25 ausgebildet. Sperrelement 10B umfasst einen Träger 26 mit einem Fortsatz 27, der bevorzugt als keilförmiger Fortsatz ausgebildet ist, was das Aufschieben des Instruments 91, 92 auf den Steckzapfen 4B erleichtert. Träger 26 und Schiebeelement 8B sind in einer gemeinsamen Bohrung 28 aufgenommen, die über eine Öffnung 29 im Mantel des Steckzapfens 4B mündet.

Kontaktiert Ende 16B von Stift 11 B Wand 23, so wird Stift 11 B in Richtung des Trägers 26 verschoben, wobei die schiefe Ebene 25 unter den Träger 26 wandert und diesen verdrängt. Fortsatz 27 bewegt sich dabei radial durch Öffnung 29 und ragt über den Mantel des Steckzapfens 4B hinaus, so dass ein auf den Steckzapfen 4B aufgeschobenes instrument 91, 92 fixiert wird. Zum Lösen des Instruments 91, 92 wird die Wirkverbindung zwischen Wand 23 und Schiebeelement 8B unterbrochen, so dass Schiebeelement 8B und Fortsatz 27 in ihre in Figur 4 dargestellten Positionen zurückkehren. Bevorzugt sind ein oder mehrere Federelemente mit dem Träger 26 und / oder dem Schiebeelement 8B verbunden, die, entsprechend den Federelementen 9, 9A der Figuren 2 und 3, nach Unterbrechung der Wirkverbindung entspannen und damit die Rückbewegung von Schiebeelement 8B und Fortsatz 27 bewirken oder unterstützen.

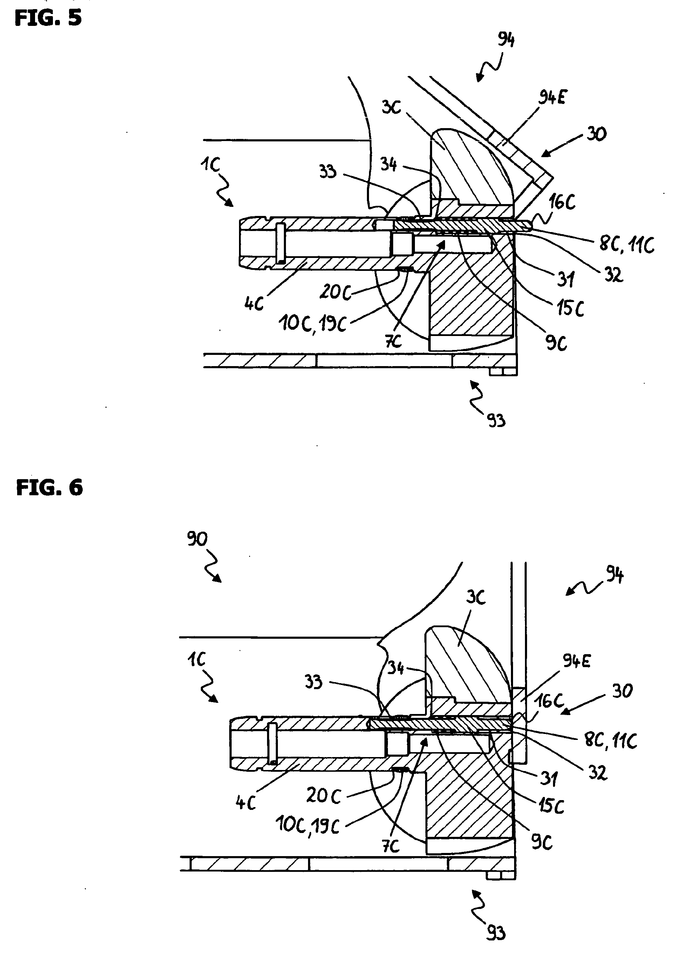

Die Figuren 5 und 6 zeigen eine Sperrvorrichtung 7C einer Anschlussvorrichtung 1C, die über den Deckel 94 der Kassette 90 betätigt wird. Deckel 94 hat hierzu eine Mitnehmervorrichtung 30, über die eine kontaktierende Wirkverbindung zum Schiebeelement 8C in Form eines Kolbens 11C hergestellt wird. Mitnehmervorrichtung 30 ist bevorzugt als Teil der Deckelfläche 94E ausgebildet. Der Vorteil dieser Sperrvorrichtung 7C liegt darin, dass bereits mit dem Schließen des Deckels 94 als Abschluss des Beladevorgangs der Reinigungskassette 90 die Instrumente 91, 92 fixiert sind und somit auch während des Einsteckens der Kassette 90 in das Reinigungs- oder Pflegegerät ein Verschieben der Instrumente 91, 92 verhindert wird.

Figur 6 zeigt die Reinigungskassette 90 mit geöffnetem Deckel 94. Eine Bohrung 31 durchsetzt Anschlussvorrichtung 1C und endet in einer Öffnung 32 an der dem Steckzapfen 4C gegenüberliegenden Seite der Anschlussvorrichtung 1C. Sperrelement 10C ist als Sprengring 19C ausgebildet und wie zu Figur 2 beschrieben in einer Ringnut 20C aufgenommen. Bohrung 31 und Ringnut 20C sind wiederum über einen Durchstich miteinander verbunden. In Bohrung 31 ist Kolben 11C beweglich aufgenommen. Kolben 11C umfasst einen Einstich 33, dessen Durchmesser geringer ist als der Durchmesser des übrigen Kolbens 11C.

In der in Figur 6 dargestellten Position der Sperrvorrichtung 7C ist Schiebeelement 8C durch die kontaktierende Wirkverbindung zwischen dem Ende 16C und dem Mitnehmer 30 vollständig in Bohrung 31 eingeschoben. In dieser Position befindet sich Einstich 33 im Bereich des Durchstichs zwischen Ringnut 20C und Bohrung 31, so dass Sprengring 19C in den Durchstich und Einstich 33 ragt und insbesondere nicht über den Mantel des Steckzapfens 4C hinaus steht. In dieser Position ist Instrument 91, 92 auf Steckzapfen 4C aufsteckbar oder von ihm abnehmbar.

Bohrung 31 umfasst einen ersten und zweiten Abschnitt mit unterschiedlichen Durchmessern, so dass dort, wo diese beiden Abschnitte aneinander stoßen, eine Schulter 34 gebildet wird. Schulter 34 dient als Lager für ein Federelement 9C. Analog zum Federelement 9 der Anschlussvorrichtung 1 in Figur 2, steht das zweite Ende des Federelements 9C mit einem Flansch 15C in Kontakt, so dass durch das Verschieben des Schiebeelements 8C Federelement 9C komprimiert wird. Wird, wie in Figur 5 dargestellt, Deckel 94 um Drehachse 96 gedreht und damit die Wirkverbindung zwischen Ende 16C von Schiebeelement 8C und dem Mitnehmer 30 aufgehoben, dann wird Schiebeelement 8C durch die Federkraft des sich entspannenden Federelements 9C in Richtung der Öffnung 32 verschoben. Dadurch entfernt sich der Abschnitt des Schiebeelements 8C mit Einstich 33 vom Durchstich zwischen Ringnut 20C und Bohrung 31 und es gelangt ein Abschnitt des Schiebeelements 8C mit einem größeren Durchmesser als Einstich 33 zum Durchstich. Der Abschnitt mit dem größeren Durchmesser verdrängt Sprengring 19 radial nach außen, so dass er über die äußere Mantelfläche des Steckzapfens 4C hinausragt und ein auf den Steckzapfen 4C aufgestecktes Instrument 91, 92 fixiert.

Sind, wie bereits zu Figur 2 beschrieben, Anschlussvorrichtung 1 bzw. 1C und / oder Träger 3 bzw. 3C dreh- oder schwenkbar ausgeführt, so kann Mitnehmervorrichtung 30 auch zur Übertragung der Drehbewegung des Deckels 94 auf die Anschlussvorrichtung 1, 1C und / oder den Träger 3, 3C verwendet werden. Dreht der Anwender Deckel 94 entsprechend der in den Figuren 5 und 6 dargestellten Drehrichtung weiter, so dreht Mitnehmervorrichtung 30 Anschlussvorrichtung 1 und / oder den Träger 3 um Drehachse 96, so dass Anschlussvorrichtung 1 bzw. 1C die in Figur 10 dargestellte Position einnimmt, in der Steckzapfen 4, 4C von Basisteil 93 weg weist. Dies erleichtert dem Anwender das Aufstecken oder Lösen der Instrumente 91, 92.

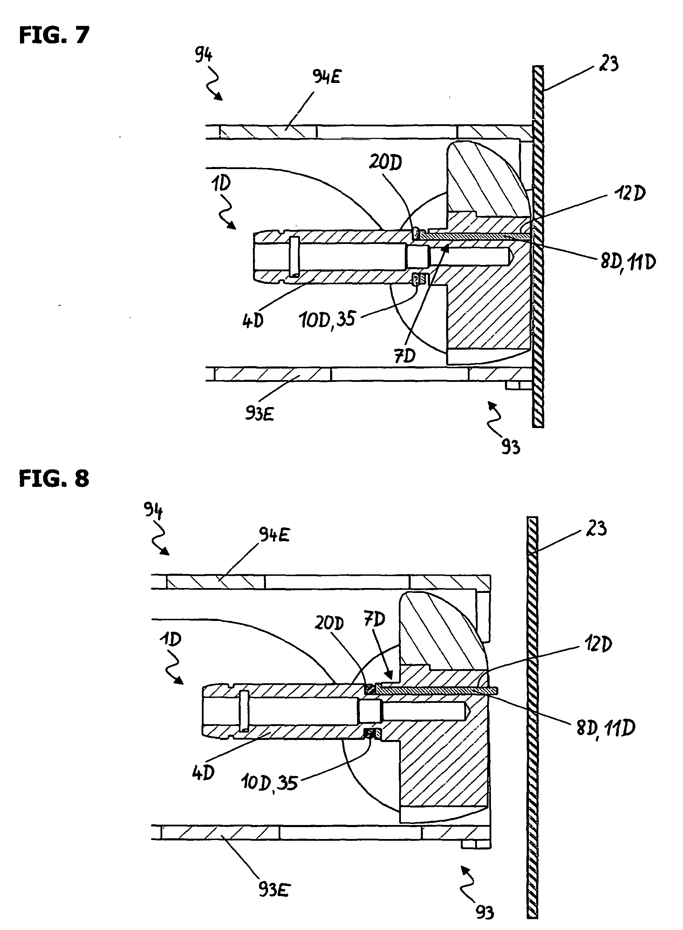

Auch die in Figur 7 und 8 dargestellte Sperrvorrichtung 7D einer Anschlussvorrichtung 1 D umfasst ein in einer Bohrung 12D aufgenommenes Schiebeelement 8D in Form eines Stifts 11D, das, wie in Zusammenhang mit Figur 3 bereits beschrieben, gemeinsam mit einem Bauteil des Reinigungs- oder Pflegegeräts, insbesondere mit der Rückwand 23 des Innenraums des Reinigungs- oder Pflegegeräts, in das die Reinigungskassette 90 eingesetzt wird, eine kontaktierende Wirkverbindung bildet. Die von der Wirkverbindung übertragene Kraft bewegt Schiebeelement 8D von einer ersten in eine zweite Position. Der Vorteil der Sperrvorrichtung 7D liegt insbesondere in ihrem einfachen Aufbau.

Figur 8 zeigt die Position, in der ein Instrument 91, 92 auf die Anschlussvorrichtung 1 D aufschiebbar oder aufsteckbar bzw. von der Anschlussvorrichtung 1 D lösbar ist. Bohrung 12D endet in einer Ringnut 20D, in der Sperrelement 10D aufgenommen ist. Sperrelement 10D ist als elastisch verformbares Element 35, zum Beispiel als O-Ring, ausgebildet, das in dieser Position nicht über die äußere Mantelfläche des Steckzapfens 4D hinausragt.

Wird, wie in Figur 7 dargestellt, Schiebeelement 8D durch Wand 23 in die Position bewegt, in der ein auf den Steckzapfen 4D aufgeschobenes Instrument 91, 92 fixiert ist, so verformt Schiebeelement 8D das elastisch verformbare Element 35, so dass es über die äußere Mantelfläche des Steckzapfens 4D hinaus steht. Für eine verbesserte, gleichmäßige Kraftübertragung weist Schiebeelement 8D an seinem dem elastisch verformbaren Element 35 zugewandten Ende eine flanschartige Verbreiterung auf, die in Ringnut 20D angeordnet ist.

Die Rückbewegung von Schiebeelement 8D nach dem Ende der kontaktierenden Wirkverbindung zwischen der Wand 23 und Schiebeelement 8D erfolgt bevorzugt allein durch die Rückstellkraft des in seine ursprüngliche, in Figur 8 dargestellte Form und Position zurückkehrende elastisch verformbaren Elements 35, kann jedoch zusätzlich auch durch ein Federelement, das mit dem Schiebeelement 8D verbunden ist, unterstützt werden.

Die Sperrvorrichtung 7E gemäß Figur 9 umfasst eine Ringnut 36, in der ein als elastisch verformbares Element 37, zum Beispiel als Membran, ausgebildetes Sperrelement 10E angeordnet ist. Eine Verbindungsbohrung 38 zwischen der Sperrvorrichtung 7E und der Medienbohrung oder -leitung 5E ermöglicht eine Ableitung eines Teils des in der Medienleitung 5E fließenden Mediums zur Sperrvorrichtung 7E. Das unter Druck stehende Medium, zum Beispiel Druckluft, Wasser, ein Schmiermittel oder eine Reinigungs- oder Pflegelösung, fließt über Verbindungsbohrung 38 in die Ringnut 36 und verformt oder dehnt das elastisch verformbare Element 37, so dass das elastisch verformbare Element 37 über die äußere Mantelfläche des Steckzapfens 4E hinausragt. Das Medium überträgt somit direkt die zur Bewegung der Sperrvorrichtung 7E von der ersten in die zweite Position benötigte Kraft auf die Sperrvorrichtung 7E. Die Sperrvorrichtung 7E nimmt damit jene Position ein, in der ein auf den Steckzapfen 4E aufgeschobenes Instrument 91, 92 fixiert ist.

Wird die Förderung des Mediums durch Medienleitung 5E beendet, so wird auch der Fluß des Mediums in die Ringnut 36 unterbrochen bzw. endet der auf das Medium ausgeübte Druck. Damit kann das elastisch verformbare Element 37 in seine ursprüngliche, nicht verformte oder gedehnte Position zurückkehren, in der es nicht über die äußere Mantelfläche des Sieckzapfens 4E hinausragt. In dieser Position kann ein Instrument 91, 92 auf den Steckzapfen 4E aufgeschoben oder von ihm abgenommen werden.

Selbstverständlich besteht auch die Möglichkeit einen direkten Kontakt zwischen dem Medium und dem Sperrelement 10E zu vermeiden, zum Beispiel wenn die Gefahr besteht, dass das Medium das Sperrelement 10E angreift oder korrodiert. In diesem Fall ist in der Verbindungsbohrung 38 oder einer weiteren Bohrung ein Schiebeelement, bevorzugt einen Stift, verschiebbar gelagert. Das zur Sperrvorrichtung abgeleitete Medium dringt in die Verbindungsbohrung 38 ein und verdrängt das Schiebeelement von einer ersten in eine zweite Position. Die Sperrvorrichtung umfasst weiters ein Sperrelement 10E, das bevorzugt als Sprengring, Fortsatz oder elastisch verformbares Element ausgebildet ist und in analoger Weise wie für die Figuren 2 - 8 beschrieben durch das Schiebeelement verschoben, verdrängt oder verformt wird, so dass es über die äußere Mantelfläche des Steckzapfens 4E hinausragt. Die Kraftübertragung durch das Medium auf das Sperrelement 10E der Sperrvorrichtung 7E erfolgt hiermit indirekt.

Der Vorteil beider Ausführungsformen der Sperrvorrichtung 7E liegt darin, dass eine Verriegelung des Instruments 91, 92 insbesondere dann gewährleistet ist, wenn durch die Medienführung Druck auf das Instrument 91, 92 ausgeübt wird.

In einem weiteren, nicht in einer Abbildung dargestellten Ausführungsbeispiel ist die Wirkverbindung als berührungslose, bevorzugt magnetische Wirkverbindung, ausgebildet. Die für die Bewegung der Sperrvorrichtung von ihrer ersten in ihre zweite Position benötigte Kraft wird hierbei durch magnetische Anziehungs- oder Abstoßungskräfte bereitgestellt. Dazu sind ein Bauteil des Reinigungs- oder Pflegegeräts oder ein Teil der Kassette 90 und zumindest ein Teil der Sperrvorrichtung magnetisiert, magnetisierbar oder mit einem Permanentmagneten versehen. Durch Annäherung des Bauteils des Reinigungs- oder Pflegegeräts oder der Kassette 90 an die Sperrvorrichtung treten die magnetischen Bauteile und / oder Permanentmagnete in Wechselwirkung. Dies kann zum Beispiel durch eine Anordnung gemäß Figur 3 erfolgen, wobei eine magnetische Wand 23 und ein magnetisches Schiebeelement 8A vorgesehen sind. Die Pole der magnetischen Wand 23 und des magnetischen Schiebeelements 8A sind dabei so angeordnet, dass gleiche Pole einander zugewandt sind, so dass bei Annäherung der Kassette 90 an die Wand 23 eine Abstoßung erfolgt, wodurch, wie zu Figur 3 bereits beschrieben, das Schiebeelement 8A in Richtung Sperrelement 10A bewegt wird. Klarerweise ist es bei einer berührungslosen Wirkverbindung nicht notwendig, dass das Ende 16A des Schiebeelements 8A aus der Bohrung 12A herausragt bzw. so weit wie bei einer kontaktierenden Wirkverbindung herausragt.

Der Vorteil einer berührungslosen Wirkverbindung liegt insbesondere darin, dass die Anordnung oder Ausformung jener Bauteile, die die Wirkverbindung bilden, weniger exakt aufeinander abgestimmt werden müssen, als dies bei einer kontaktierenden Wirkverbindung notwendig ist, damit bei letzterer garantiert ist, dass die Kraftübertragung sicher und ausreichend erfolgt. Insbesondere besteht bei berührungslosen Wirkverbindungen die Möglichkeit nur einen Typ von Reinigungskassette 90 für verschiedene Reinigungs- oder Pflegegerät herzustellen, ohne gezwungen zu sein den Bereich der Wirkverbindung des Reinigungs- oder Pflegegeräts und der Kassette 90 exakt aufeinander abstimmen zu müssen.

## Patentansprüche

1. Reinigungs- oder Pflegegerät oder Reinigungskassette (90) mit einer Anschlussvorrichtung (1 - 1D) für medizinische Instrumente (91, 92), wobei die Anschlussvorrichtung (1 - 1 D) einen Steckzapfen (4 - 4D) zum Aufschieben oder Aufstecken des Instruments (91, 92), zumindest eine Medienleitung (5, 5A) zur Verbindung einer Quelle eines Reinigungs- oder Pflegemediums mit dem Inneren des Instruments (91, 92) und eine Sperrvorrichtung (7 - 7D) zur Fixierung des Instruments (91, 92) an der Anschlussvorrichtung (1 - 1D)aufweist, so dass während der Reinigung oder Pflege das unter Druck stehende Reinigungs- oder Pflegemedium das Instrument (91, 92) nicht verschiebt oder von der Anschlussvorrichtung (1 - 1D) löst, wobei die Sperrvorrichtung (7 - 7D) relativ zur Anschlussvorrichtung (1 - 1 D) zwischen einer ersten Position und einer zweiten Position bewegbar ist, und in einer der beiden Positionen das Instrument (91, 92) auf die Anschlussvorrichtung (1 - 1D) aufschiebbar oder aufsteckbar bzw. von der Anschlussvorrichtung (1 - 1 D) lösbar ist und in der anderen Position das Instrument (91, 92) an der Anschlussvorrichtung (1 - 1 D) fixierbar ist, **gekennzeichnet durch**
eine direkte oder indirekte Wirkverbindung zwischen der Sperrvorrichtung (7 - 7D) und einem Bauteil des Reinigungs- oder Pflegegeräts oder zwischen der Sperrvorrichtung (7 - 7D) und zumindest einem Teil der Kassette (90) zur Übertragung der zur Bewegung der Sperrvorrichtung (7 - 7D) von der ersten Position in die zweite Position benötigten Kraft auf die Sperrvorrichtung (7 - 7D).

2. Reinigungs- oder Pflegegerät oder Reinigungskassette (90) nach Anspruch 1,
**gekennzeichnet durch**
eine berührungslose Wirkverbindung, insbesondere eine magnetische Wirkverbindung, bei der zumindest Teile der Sperrvorrichtung (7 - 7D) und des Bauteils des Reinigungs-oder Pflegegeräts oder der Kassette (90) magnetisch sind.

3. Reinigungs- oder Pflegegerät oder Reinigungskassette (90) nach Anspruch 1,
**gekennzeichnet durch**
eine kontaktierende Wirkverbindung, **durch** die zwischen einem Bauteil des Reinigungs-oder Pflegegeräts oder einem Teil der Kassette (90) und der Sperrvorrichtung (7 - 7D) ein Kontakt herstellbar ist.

4. Reinigungs- oder Pflegegerät oder Reinigungskassette (90) nach Anspruch 3, **dadurch**
**gekennzeichnet, dass**
die Sperrvorrichtung (7 - 7D) ein Schiebeelement (8 - 8D) umfasst, auf das die zur Bewegung benötigte Kraft durch die Wirkverbindung übertragbar ist.

5. Reinigungs- oder Pflegegerät oder Reinigungskassette (90) nach Anspruch 4, **dadurch**
**gekennzeichnet, dass**
das Schiebeelement (8 - 8D) als Stift (11, 11 B, 11D) oder als Kolben (11A, 11 C) ausgebildet ist, der bevorzugt durch ein Federelement (9, 9A, 9C) vorgespannt ist.

6. Reinigung- oder Pflegegerät oder Reinigungskassette (90) nach Anspruch 4 oder 5,
**dadurch gekennzeichnet, dass**
das Schiebeelement (8 - 8D) eine schiefe Ebene (25) oder einen Einstich (33) umfasst.

7. Reinigungs- oder Pflegegerät oder Reinigungskassette (90) nach einem der vorhergehenden Ansprüche, **dadurch gekennzeichnet, dass**
die Sperrvorrichtung (7 - 7D) ein Sperrelement (10 - 10D) umfasst, das radial verschiebbar oder verformbar am Steckzapfen (4 - 4D) angeordnet ist, wobei das Sperrelement (10 - 10D) bevorzugt als Sprengring (19, 19A, 19C), Fortsatz (27) oder elastisch verformbares Element (35) ausgebildet ist.

8. Reinigungs- oder Pflegegerät oder Reinigungskassette (90) nach einem der vorhergehenden Ansprüche, **dadurch gekennzeichnet, dass**
die Anschlussvorrichtung (1 - 1 D) drehbar ausgebildet ist.

9. Reinigungs- oder Pflegegerät oder Reinigungskassette (90) nach einem der vorhergehenden Ansprüche, **dadurch gekennzeichnet, dass**
die Anschlussvorrichtung (1 - 1 D) in einer Reinigungskassette (90) mit einem drehbaren Deckel (94) angeordnet ist und der Deckel (94) eine Mitnehmervorrichtung (30) umfasst, durch die zumindest ein Teil der Drehbewegung des Deckels (94) auf die Anschlussvorrichtung (1 - 1D) übertragbar ist.

10. Reinigungs- oder Pflegegerät oder Reinigungskassette (90) nach Anspruch 9, **dadurch**
**gekennzeichnet, dass**
die Anschlussvorrichtung (1 - 1 D) und der Deckel (94) eine gemeinsame Drehachse (96) haben, die bevorzugt durch eine einzige Welle (97) gebildet ist.

11. Anschlussvorrichtung (1E) für ein Reinigungs- oder Pflegegerät oder eine Reinigungskassette (90) mit einem Steckzapfen (4E) zum Aufschieben oder Aufstecken des Instruments (91, 92), zumindest einer Medienleitung (5E) zur Verbindung einer Quelle eines Reinigungs- oder Pflegemediums mit dem Inneren des Instruments (91, 92) und einer Sperrvorrichtung (7E) zur Fixierung des Instruments an der Anschlussvorrichtung (1 E), so dass während der Reinigung oder Pflege das unter Druck stehende Reinigungs- oder Pflegemedium das Instrument nicht verschiebt oder von der Anschlussvorrichtung (1 E) löst, wobei die Sperrvorrichtung (7E) relativ zur Anschlussvorrichtung (1 E) zwischen einer ersten Position und einer zweiten Position bewegbar ist, und in einer der beiden Positionen das Instrument (91, 92) auf die Anschlussvorrichtung (1 E) aufschiebbar oder aufsteckbar bzw. von der Anschlussvorrichtung (1 E) lösbar ist und in der anderen Position das Instrument (91, 92) an der Anschlussvorrichtung (1 E) fixierbar ist, **gekennzeichnet durch**
eine Verbindungsbohrung (38) zwischen der Sperrvorrichtung (7E) und der Medienleitung (5E) zur Abteilung eines Teils des in der Medienleitung (5E) fließenden und unter Druck stehenden Medium, so dass die zur Bewegung der Sperrvorrichtung (7E) von der ersten in die zweite Position benötigte Kraft **durch** das Medium direkt oder indirekt auf die Sperrvorrichtung (7E) übertragbar ist.

12. Anschlussvorrichtung (1 E) nach Anspruch 11, **dadurch gekennzeichnet, dass**
die Sperrvorrichtung (7E) ein Schiebeelement, bevorzugt einen Stift, umfasst, wobei das Schiebeelement durch Verdrängung durch das zur Sperrvorrichtung (7E) abgeleitete Medium von einer ersten in eine zweite Position bewegbar ist.

13. Anschlussvorrichtung (1 E) nach Anspruch 11 oder 12, **dadurch gekennzeichnet, dass**
die Sperrvorrichtung (7E) ein Sperrelement umfasst, das radial verschiebbar oder verformbar am Steckzapfen (4E) angeordnet ist, wobei das Sperrelement bevorzugt als Sprengring, Fortsatz oder elastisch verformbares Element ausgebildet ist.

14. Anschlussvorrichtung (1 E) nach Anspruch 11, **dadurch gekennzeichnet, dass**
die Sperrvorrichtung (7E) ein elastisch verformbares Element (37), bevorzugt eine Membran umfasst, wobei das elastisch verformbare Element (37) durch direkten Kontakt mit dem durch die Verbindungsbohrung (38) abgeleiteten Medium verformbar ist.

## Claims

1. Cleaning or care apparatus or cleaning cassette (90) having a connecting device (1 - 1 D) for medical instruments (91, 92), wherein the connecting device (1 - 1 D) comprises a plug pin (4 - 4D) for attaching or connecting the instrument (91, 92), at least one media line (5, 5A) for connecting a source of a cleaning or care medium to the interior of the instrument (91, 92) and a blocking device (7 - 7D) for securing the instrument (91, 92) on the connecting device (1 - 1 D), so that the cleaning or care medium which is under pressure during the cleaning or care operation does not displace the instrument (91, 92) or release it from the connecting device (1 - 1 D), wherein the blocking device (7 - 7D) is movable between a first position and a second position in relation to the connecting device (1 - 1D), and in one of the two positions, the instrument (91, 92) can be attached or connected to the connecting device (1 - 1D) or released from the connecting device (1
- 1D) and in the other position the instrument (91, 92) can be attached to the connecting device (1 - 1 D), **characterized by**
a direct or indirect operative connection between the blocking device (7 - 7D) and a component of the cleaning or care apparatus or between the blocking device (7 - 7D) and at least a part of the cassette (90) for transmitting to the blocking device (7 - 7D) the force required to move the blocking device (7 - 7D) from the first position into the second position.

2. Cleaning or care apparatus or cleaning cassette (90) according to claim 1, **characterized by**
a non - contacting operative connection, in particular a magnetic operative connection, in which at least parts of the blocking device (7 - 7D) and the component of the cleaning or care apparatus or the cassette (90) are magnetic.

3. Cleaning or care apparatus or cleaning cassette (90) according to claim 1, **characterized by**
a contacting operative connection which can establish a contact between a component of the cleaning or care apparatus or a part of the cassette (90) and the blocking device (7 - 7D).

4. Cleaning or care apparatus or cleaning cassette (90) according to claim 3, **characterized in that**
the blocking device (7 - 7D) comprises a shift element (8 - 8D) to which the force required for the movement can be transmitted by way of the operative connection.

5. Cleaning or care apparatus or cleaning cassette (90) according to claim 4, **characterized in that**
the shift element (8 - 8D) is designed as a pin (11, 11B, 11D) or as a piston (11A, 11C) which is preferably prestressed by a spring element (9, 9A, 9C).

6. Cleaning or care apparatus or cleaning cassette (90) according to claim 4 or 5,
**characterized in that**
the shift element (8 - 8D) comprises an inclined plane (25) or a recess (33).

7. Cleaning or care apparatus or cleaning cassette (90) according to any of the preceding claims, **characterized in that**
the blocking device (7 - 7D) comprises a blocking element (10 - 10D) which is arranged radially displaceable or deformable on the plug pin (4 - 4D), wherein the blocking element (10 - 10D) is preferably designed as a spring ring (19, 19A, 19C), a protrusion (27) or an elastically deformable element (35).

8. Cleaning or care apparatus or cleaning cassette (90) according to any of the preceding claims, **characterized in that**
the connecting device (1 - 1 D) is designed to be rotatable.

9. Cleaning or care apparatus or cleaning cassette (90) according to any of the preceding claims, **characterized in that**
the connecting device (1 - 1 D) is arranged in a cleaning cassette (90) with a rotatable cover (94), and the cover (94) comprises an entraining device (30) so that at least a portion of the rotational movement of the cover (94) can be transferred to the connecting device (1 - 1 D).

10. Cleaning or care apparatus or cleaning cassette (90) according to claim 9, **characterized in that**
the connecting device (1 - 1 D) and the cover (94) have a shared axis of rotation (96) which is preferably formed by a single shaft (97).

11. Connecting device (1E) for a cleaning or care apparatus or a cleaning cassette (90) with a plug pin (4E) for attaching or connecting the instrument (91, 92), at least one media line (5E) for connecting a source of a cleaning or care medium to the interior of the instrument (91, 92) and a blocking device (7E) for securing the instrument on the connecting device (1 E) so that during the cleaning or care operation, the cleaning or care medium which is under pressure does not cause the instrument to shift or be released from the connecting device (1 E), wherein the blocking device (7E) is movable in relation to the connecting device (1 E) between a first position and a second position, and in one of the two positions, the instrument (91, 92) can be attached or connected to the connecting device (1 E) or released from the connecting device (1E) and in the other position the instrument (91, 92) can be attached to the connecting device (1E), charactericed by
a connecting bore (38) between the blocking device (7E) and the media line (5E) for draining off a portion of the medium which flows in the media line (5E) and is under pressure, so that the force required to move the blocking device (7E) from the first position into the second position can be transmitted by the medium directly or indirectly to the blocking device (7E).

12. Connecting device (1 E) according to claim 11, **characterized in that**
the blocking device (7E) comprises a shift element, preferably a pin, and wherein the shift element is movable from a first position into a second position by displacement by means of the medium which is drained out to the blocking device (7E).

13. Connecting device (1 E) according to claim 11 or 12, **characterized in that**
the blocking device (7E) comprises a blocking element that is arranged radially displaceable or deformable on the plug pin (4E) and wherein the blocking element is preferably designed as a spring ring, protrusion or elastically deformable element.

14. Connecting device (1 E) according to claim 11, **characterized in that**
the blocking device (7E) comprises an elastically deformable element (37), preferably a membrane, and wherein the elastically deformable element (37) is deformable by direct contact with the medium drained out through the connecting bore (38).

## Revendications

1. Appareil de nettoyage ou d'entretien ou encore cassette de nettoyage (90), avec un dispositif de raccordement (1 - 1 D) pour des instruments médicaux (91, 92), dans lequel le dispositif de raccordement (1 - 1D) comporte un pivot d'enfichage (4 - 4D) permettant d'enfoncer ou d'enficher l'instrument (91, 92), au moins une conduite de fluide (5, 5A) pour relier une source d'un fluide de nettoyage ou d'entretien avec l'intérieur de l'instrument (91, 92), et un dispositif de blocage (7 - 7D) pour fixer l'instrument (91, 92) au dispositif de raccordement (1 - 1 D), de façon à ce que le fluide de nettoyage ou d'entretien sous pression ne fasse se déplacer ou se détacher l'instrument (91, 92) du dispositif de raccordement (1 - 1 D) pendant le nettoyage ou l'entretien, le dispositif de blocage (7 - 7D) pouvant se déplacer par rapport au dispositif de raccordement (1 - 1 D) entre une première position et une deuxième position, l'instrument (91, 92) pouvant être enfoncé ou enfiché sur le dispositif de raccordement (1 - 1 D) dans l'une des deux positions et l'instrument (91, 92) pouvant être fixé au dispositif de raccordement (1 - 1D) dans l'autre position, **caractérisé en ce que**
une liaison fonctionnelle directe ou indirecte entre le dispositif de blocage (7 - 7D) et un élément de l'appareil de nettoyage ou d'entretien ou encore entre le dispositif de blocage (7 - 7D) et au moins une partie de la cassette (90) est prévue pour transmettre au dispositif de blocage (7 - 7D) la force nécessaire au déplacement du dispositif de blocage (7 - 7D) de la première position vers la deuxième position.

2. Appareil de nettoyage ou d'entretien ou cassette de nettoyage (90) selon la revendication 1,
**caractérisé en ce que**
une liaison fonctionnelle sans contact, notamment une liaison fonctionnelle magnétique, est prévue, dans laquelle au moins une partie du dispositif de blocage (7 - 7D) et de l'élément de l'appareil de nettoyage ou d'entretien ou de la cassette (90) sont magnétiques.

3. Appareil de nettoyage ou d'entretien ou cassette de nettoyage (90) selon la revendication 1,
**caractérisé en ce que**
une liaison fonctionnelle avec contact permet de réaliser un contact entre un élément de l'appareil de nettoyage ou d'entretien ou une partie de la cassette (90) et le dispositif de blocage (7 - 7D).

4. Appareil de nettoyage ou d'entretien ou cassette de nettoyage (90) selon la revendication 3
**caractérisé en ce que**
le dispositif de blocage (7 - 7D) comprend un poussoir (8 - 8D), auquel peut être transmise par la liaison fonctionnelle la force nécessaire au déplacement.

5. Appareil de nettoyage ou d'entretien ou cassette de nettoyage (90) selon la revendication 4
**caractérisé en ce que**
le poussoir (8 - 8D) est conçu comme une tige (11, 11 B, 11 D) ou comme un piston (11 A, 11 C), qui sont préférentiellement précontraints par un élément de ressort (9, 9A, 9C).

6. Appareil de nettoyage ou d'entretien ou cassette de nettoyage (90) selon les revendications 4 ou 5,
**caractérisé en ce que** le poussoir (8 - 8D) comprend une surface inclinée (25) ou une encoche (33).

7. Appareil de nettoyage ou d'entretien ou cassette de nettoyage (90) selon l'une des revendications précédentes,
**caractérisé en ce que**
le dispositif de blocage (7 - 7D) comprend un élément de blocage (10 - 10D), qui est disposé sur le pivot d'enfichage (4 - 4D) de façon à pouvoir être radialement déplacé ou déformé, l'élément de blocage (10 - 10D) étant préférentiellement conçu comme une bague élastique 19 (19, 19A, 19C), un prolongement (27) ou un élément élastiquement déformable (35).

8. Appareil de nettoyage ou d'entretien ou cassette de nettoyage (90) selon l'une des revendications précédentes,
**caractérisé en ce que**
le dispositif de raccordement (1 - 1 D) est conçu de manière rotative.

9. Appareil de nettoyage ou d'entretien ou cassette de nettoyage (90) selon l'une des revendications précédentes,
**caractérisé en ce que**
le dispositif de raccordement (1 - 1 D) est disposé dans une cassette de nettoyage (90) avec un couvercle rotatif (94) et **en ce que** le couvercle (94) comprend un dispositif d'entraînement (30), par lequel au moins une partie du mouvement de rotation du couvercle (94) peut être transmise au dispositif de raccordement (1- 1D).

10. Appareil de nettoyage ou d'entretien ou cassette de nettoyage (90) selon la revendication 9,
**caractérisé en ce que**
le dispositif de raccordement (1 - 1D) et le couvercle (94) ont un axe de rotation (96) commun, qui est préférentiellement constitué d'un seul arbre (97).

11. Dispositif de raccordement (1 E) pour un appareil de nettoyage ou d'entretien ou une cassette de nettoyage (90), avec un pivot d'enfichage (4E) pour l'enfoncement ou l'enfichage de l'instrument (91, 92), au moins une conduite de fluide (5E) pour relier une source d'un fluide de nettoyage ou d'entretien à l'intérieur de l'instrument (91, 92) à un dispositif de blocage (7E) pour fixer l'instrument au dispositif de raccordement (1 E), de sorte que le fluide de nettoyage ou d'entretien sous pression ne déplace ou ne détache du dispositif de raccordement (1 E) l'instrument pendant le nettoyage ou l'entretien, le dispositif de blocage (7E) pouvant être déplacé par rapport au dispositif de raccordement (1 E) entre une première position et une deuxième position, l'instrument (91, 92) pouvant être enfoncé ou enfiché sur le dispositif de raccordement (1E) ou encore retiré du dispositif de raccordement (1E) dans l'une des deux positions, et l'instrument (91, 92) pouvant être fixé au dispositif de raccordement (1E) dans l'autre position,
**caractérisé en ce que**
un perçage de liaison (38) est prévu entre le dispositif de blocage (7E) et la conduite de fluide (5E) pour l'évacuation d'une partie du fluide sous pression qui s'écoule dans la conduite de fluide (5E), de façon ce que le fluide puisse transmettre directement ou indirectement au dispositif de blocage (7E) la force nécessaire au déplacement du dispositif de blocage (7E) d'une première position vers une deuxième position.

12. Dispositif de raccordement (1 E) selon la revendication 11,
**caractérisé en ce que**
le dispositif de blocage (7E) comprend un poussoir, préférentiellement une tige, le poussoir pouvant être déplacé d'une première vers une deuxième position en étant repoussé par le fluide évacué vers le dispositif de blocage (7E).

13. Dispositif de raccordement (1 E) selon les revendications 11 ou 12,
**caractérisé en ce que**
le dispositif de blocage (1 E) comprend un élément de blocage, qui peut être déformé ou déplacé radialement sur le pivot d'enfichage (4E), l'élément de blocage étant préférentiellement conçu comme une bague élastique, un prolongement ou encore un élément élastiquement déformable.

14. Dispositif de raccordement (1 E) selon la revendication 11,
**caractérisé en ce que**
le dispositif de blocage (7E) comprend un élément élastiquement déformable (37), préférentiellement une membrane, l'élément élastiquement déformable (37) pouvant être déformé par un contact direct avec le fluide évacué par le perçage de liaison (38).
